# FASCICULE DE BREVET EUROPEEN

(11) **EP 2 585 491 B1**
(45) Date de publication et mention de la délivrance du brevet: **14.05.2014**
(21) Numéro de dépôt: 11728665.8
(22) Date de dépôt: 10.06.2011
(51) Int. Cl.: C08F 2/22

(54) **EMULSIONS ACRYLIQUES ALKALI GONFLABLES SANS TENSIO ACTIF, LEUR UTILISATION DANS DES FORMULATIONS AQUEUSES ET FORMULATIONS LES CONTENANT**
TENSIDFREIE, IN ALKALISCHEN MEDIEN QUELLFÄHIGE ACRYLEMULSIONEN, DEREN VERWENDUNG IN WÄSSRIGEN FORMULIERUNGEN UND FORMULIERUNGEN, DIE DIESE ENTHALTEN
SURFACTANT-FREE ALKALI-SWELLABLE ACRYLIC EMULSIONS, USE THEREOF IN AQUEOUS FORMULATIONS AND FORMULATIONS CONTAINING SAME

(30) Priorité: 25.06.2010 FR 1055080
(43) Date de publication de la demande: 01.05.2013
(73) Titulaire: Coatex S.A.S, 69730 Genay (FR)
(72) Inventeur: SUAU, Jean-Marc, F-69480 Lucenay (FR)
(74) Mandataire: Fiorucci, Hélène
(86) Numéro de dépôt international: PCT/IB2011/001341
(87) Numéro de publication internationale: WO 2011/161511

(56) Documents cités:
- EP-A1- 0 562 344
- EP-A1- 1 777 241
- WO-A1-2004/063228

## Description

La présente invention se rapporte à de nouvelles émulsions épaississantes alkali gonflables, exemptes de tensio-actif et de solvant autre que l'eau : on évite ainsi tous les inconvénients liés à l'emploi de solvants, ou à la formation de mousse dans le cas de tensio-actifs. Ces nouvelles émulsions contiennent une certaine quantité d'acide 2-acrylamido-2-méthylpropane sulfonique (ou AMPS, n° CAS : 40623-75-4). Elles s'avèrent efficaces pour épaissir des milieux aqueux, notamment des peintures aqueuses, des sauces de couchage papetière, des suspensions aqueuses de matières minérales, des détergents, des formulations cosmétiques, ou des formulations contenant un liant hydraulique.

Contrôler la rhéologie des formulations aqueuses contenant éventuellement des charges minérales est une nécessité, tant au stade de la fabrication de ces produits, que pendant leur transport, leur stockage ou au cours de leur mise en oeuvre. La diversité des contraintes pratiques au niveau de chacune de ces étapes renvoie à une multiplicité de comportements rhéologiques. Dans le cas d'une peinture par exemple, on peut résumer le besoin de l'homme du métier à l'obtention d'un effet d'épaississement de celle-ci, tant pour des raisons de stabilité au cours du temps, que pour une possible application de la peinture sur une surface verticale, l'absence d'éclaboussures au moment de la mise en oeuvre, ou de coulure après la mise en oeuvre.

De fait, on a désigné les produits qui contribuent à cette régulation du comportement rhéologique sous le terme d'épaississants. Présents dans le secteur des peintures aqueuses, on les trouve également dans les sauces de couchage papetières, les suspensions de matières minérales, les détergents, les formulations cosmétiques, ou encore les bétons et les ciments. Parmi ces épaississants, l'homme du métier connaît depuis longtemps la catégorie particulière des émulsions acryliques alkali gonflables, qui sont des polymères en émulsion directe dans l'eau à partir de tensio-actifs, lesdits polymères étant constitués d'au moins un monomère non hydrosoluble et d'au moins un monomère hydrosoluble alkali gonflable dont l'acide méthacrylique.

La Demanderesse précise que l'expression « émulsion directe d'un polymère dans l'eau » désigne une dispersion stable et homogène de particules de polymère dans l'eau (on ne fait pas référence ici aux émulsions du type huile dans eau ou eau dans huile, qui impliquent l'existence de deux phases distinctes, l'une aqueuse et l'autre huileuse). Quant à l'expression « polymère alkali gonflable », elle signifie que ledit polymère est capable, lorsque le milieu est alcalin, d'incorporer une quantité d'eau telle qu'il y a formation d'un gel et donc augmentation de la viscosité.

Il existe 2 grandes familles d'épaississants acryliques alkali gonflables : les épaississants de type ASE (Alkali Swellable Emulsion ou émulsion alcali gonflable) et ceux de type HASE (Hydrophobically modified Alkali Swellable Emulsion ou émulsion alkali gonflable et modifiée hydrophobiquement). Les premiers désignent des copolymères de l'acide méthacrylique avec un ester non hydrosoluble de cet acide, et les seconds désignent des copolymères à base d'acide méthacrylique, d'un ester non hydrosoluble de l'acide (méth)acrylique et d'un monomère disposant de groupements hydrophobes dits « associatifs ». Ces copolymères peuvent en outre être réticulés.

Les mécanismes d'action de ces produits diffèrent. Les polymères de type ASE n'épaississent qu'à l'état neutralisé, d'où l'expression « alkali-gonflables » : on induit alors un mécanisme de répulsion ionique entre les différents groupements carboxylates portés par la chaîne polymérique. Ces groupements ionisés polarisent les molécules d'eau ce qui provoque l'augmentation de la viscosité du milieu. En plus du phénomène ionique précité, les polymères de type HASE mettent en jeu des interactions entre leurs groupements hydrophobes associatifs, ce qui contribue aussi à épaissir le milieu. Ces mécanismes, et notamment le caractère alkali gonflable de ces émulsions et leur capacité à épaissir un milieu aqueux à un pH voisin de la neutralité, ont été décrits dans les documents WO 2007 / 144721 et « Practical guide to associative thickeners » (Proceedings of the Annual Meeting Technical Program of the FSCT, 2000, 78th, 644-702).

On trouve de nombreuses applications de ces épaississants dans la peinture, les sauces de couchage, ou la cosmétique (voir les demandes de brevet FR 2 693 203 A1, FR 2 872 815 A1, FR 2 633 930 A1, FR 2 872 815 A1). En outre, ils existent sous forme commerciale, notamment à travers les gammes de produits Rheocarb™, Rheocoat™, Thixol™, Rheotech™, Polyphobe™ et Viscoatex™ commercialisées par la société COATEX™ S.A.S.

De manière générale, les épaississants de type ASE et HASE sont fabriqués sous forme d'émulsions directes du polymère alkali gonflable dans l'eau, dont la teneur en produit actif oscille entre 10 % et 45 % de leur poids total.

Le procédé de synthèse correspondant est notamment décrit dans les publications suivantes : « Synthesis of an alkali-swellable emulsion and its effect on the rate of polymer diffusion in poly(vinyl acetate-butyl acrylate) latex films » (Journal of Polymer Science, Part A: Polymer Chemistry, 2005, 43 (22), pp. 5632-5642), « Structural and rheological properties of hydrophobically modified alkali-soluble emulsion solutions » (Journal of Polymer Science, Part B: Polymer Physics, 2002, 40(18), pp. 1985-1994), « Viscoelastic properties of hydrophobically modified alkali-soluble emulsion in salt solutions » (Polymer, 1999, 40 (23), pp. 6369-6379), « Dissolution behavior in water of a model hydrophobic alkali-swellable emulsion polymer with C20H41 groups « (Canadian Journal of Chemistry, 1998, 76 (11), pp. 1779-1787).

Il fait également l'objet de nombreuses demandes de brevets (EP 0 089 213 A1, EP 0 646 606 A1, EP 0 979 833 A1 pour les ASE et EP 0 013 836 A1, WO 93 / 2454 A1, US 4 268 641 A1, US 4 421 902 A1, US 3 915 921 A1 pour les HASE).

Une constante de leur procédé de fabrication réside dans la mise en oeuvre d'agents tensio-actifs, dont la fonction première est de stabiliser les particules de polymère en suspension dans l'eau. Des tensio-actifs bien connus pour cet emploi sont le sodium lauryl sulfate, le dodécylbenzène sulfonate ou encore les sulfates d'alcools gras éthoxylés. Si on décrivait leur mise en oeuvre il y a déjà 30 ans (voir le document EP 0 013 836 déjà cité, page 7, lignes 3-12), celle-ci apparaît encore présente dans des documents bien plus récents, comme les publications scientifiques énumérées plus haut.

L'utilisation de tensio-actifs apparaît donc comme une caractéristique incontournable dans les procédés de fabrication d'émulsions de type ASE et HASE. Or, on a tendance à oublier ou négliger les inconvénients engendrés par ces tensio-actifs, dans la mesure où on estime qu'on ne peut pas se dispenser de leur emploi. Ces problèmes sont avant tout liés à la formation naturelle de mousse, dès l'instant où le milieu contenant le tensio-actif est agité : outre des inconvénients esthétiques, cette mousse peut dégrader l'efficacité de l'épaississant.

Dans le cas d'une peinture, elle peut créer des inhomogénéités au sein de la formulation aqueuse, et même altérer les propriétés du produit final c'est-à-dire du film sec de peinture résultant du séchage de la formulation aqueuse. On peut ainsi observer la formation de « cratères » ou de particules insolubles qui sont autant d'hétérogénéités nuisant à l'aspect esthétique et aux propriétés de surface du film (aspect mécanique, mais aussi propriétés optiques et état de surface). Enfin, il est bien connu que la présence de tensio-actifs dans une formulation de peinture va dégrader au final le caractère lessivable du film sec (voir « Effect of surfactants used for binder synthesis on the properties of latex paints », Progress in Organic Coatings, 2005, 53 (2), pp. 112-118).

Afin de remédier à de tels inconvénients, la Demanderesse a mis au point un nouveau procédé de fabrication d'émulsions de type ASE ou HASE, ne mettant pas en jeu de tensio-actif, ni de solvant autre que l'eau. La caractéristique principale de ce procédé est qu'il met en oeuvre, outre les monomères habituels à polymériser dans le cas des émulsions ASE et HASE, une certaine quantité d'un monomère particulier qui est l'acide 2-acrylamido-2-méthylpropane sulfonique (ou AMPS, n° CAS : 40623-75-4). De manière tout à fait surprenante et avantageuse, on parvient ainsi à fabriquer de véritables émulsions de type ASE et HASE, présentant un extrait sec commercial (supérieur à 25 % en poids sec de matière active), stables dans le temps, caractérisées par des tailles de particules similaires aux émulsions de l'art antérieur, et dont l'efficacité épaississante est avérée.

En outre, on savait jusqu'alors polymériser en émulsion de l'AMPS avec des monomères (méth)acryliques et leurs esters, mais cette technique avait toujours été décrite en présence de tensio-actifs (voir par exemple le document US 3 931 087 A1). On trouve d'autres exemples de copolymères contenant un acide carboxylique (comme l'acide acrylique) avec l'AMPS, mais toujours en présence de tensio-actif ou de solvant autre que l'eau ; or, il est clair que les solvants présentent aujourd'hui de très nombreux inconvénients à l'égard de la préservation de l'environnement. Des exemples de tels produits existent dans le domaine du ciment (EP 1 886 980 A1), de la pharmacie (EP 1 400 564 A1), de la céramique (EP 1 262 463 A1), sans qu'il s'agisse pour autant d'émulsions alkali-gonflables, encore moins d'émulsions alkali-gonflables présentant un caractère associatif.

Dans le domaine des émulsions alkali gonflables, l'AMPS avait déjà été envisagé comme co-monomère ; en revanche, personne n'avait alors pensé que les tensio-actifs n'étaient pas nécessaires à la synthèse du polymère le contenant, et personne n'avait su déceler l'influence que cela pourrait avoir sur la fabrication des émulsions de type ASE et HASE.

A titre indicatif, les documents WO 03 / 012004 A1 et WO 03 / 012004 A1 décrivent des émulsions épaississantes de type HASE incorporant de l'AMPS, mais dont la synthèse est réalisée en présence de tensio-actif et/ou de solvant autre que l'eau. Les documents FR 2 873 126 Al et FR 2 782 086 A1 décrivent quant à eux des émulsions inverses H/E réalisées avec des agents émulsionnant, d'un copolymère de l'AMPS avec de l'acide (méth)acrylique et un autre monomère qui peut être un ester de ces acides.

Enfin, toute la littérature portant sur la fabrication d'émulsions sans tensio-actifs ne pouvait orienter l'homme du métier sur la mise en oeuvre de l'AMPS. En effet, elle était avant tout centrée sur des procédés particuliers comme les ondes acoustiques, le CO₂ super-critique (« Surfactant-free emulsions », Current opinion in Colloidal and Interface Science, 13, 2008, pp. 228-235), la polymérisation radicalaire contrôlée (« Surfactant-Free, Controlled/Living Radical Emulsion Polymerization in batch conditions using a low molar mass RAFT Agent », Macromolecules, 2008, 41 (21), pp 7850-7856), ou en mini-émulsion (« Emulsifier-free miniemulsion polymerization of styrene and the investigation of encapsulation of nanoparticles with polystyrene via this procedure using an anionic initiator », Journal of Applied Polymer Science, 105, 3, pp. 1244 - 1250). Il va sans dire que le procédé objet de la présente invention est d'une simplicité sans commune mesure avec les techniques énumérées ci-dessus.

Le document WO 2004/063228 décrit des émulsions aqueuses de polymères acryliques contenant de l'AMPS préparées en présence de solvant. Les émulsions contiennent du tensio-actif.

Le document EP 0 562 344 décrit la préparation de polymères similaires aux présents polymères mais contenant une quantité trop importante d'unités issues de l'AMPS et préparées en solution.

Le document EP 1 777 241 décrit la polymérisation en émulsion de monomères en absence de tensio-actif et cite parmi les possibles monomères l'AMPS. Cependant les polymères sont différents des présents polymères et sont principalement basés sur des esters vinyliques.

Par conséquent, les polymères de type ASE et HASE qui résultent du procédé selon l'invention, et qui sont sous forme d'émulsions directes exemptes de tensio-actif et de solvant autre que l'eau, sont eux-aussi nouveaux et présentent l'avantage de ne pas engendrer de mousse sous agitation, à l'inverse de leurs prédécesseurs.

Un autre objet de la présente invention réside en l'utilisation des émulsions précitées comme agent épaississant dans des formulations aqueuses, celles-ci constituant le dernier objet de la présente invention.

Aussi, un premier objet de la présente invention consiste en un procédé de fabrication en émulsion aqueuse directe d'un polymère, caractérisé en ce que ledit procédé met en jeu la réaction de polymérisation de, exprimé en % en poids de chacun des monomères :
a) 20 % à 60 % en poids d'acide méthacrylique et éventuellement d'acide acrylique,
b) 40 % à 80 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
   le total a) + b) + c) + d) étant égal à 100 %,
ou
a) 20 % à 60 % en poids d'acide méthacrylique et éventuellement d'acide acrylique,
b) 40 % à 80 % en poids d'au moins un ester de l'acide (méth)acrylique,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
   le total a) + b) + c) + d) + e) étant égal à 100 %,
et caractérisé en ce que ledit procédé est exempt de tensio-actif et de solvant autre que l'eau.

Les autres caractéristiques de ce procédé (température, choix du système catalytique, mise en oeuvre d'un agent de transfert, emploi d'un éventuel réticulant) sont celles décrites dans l'état de la technique, notamment à travers les documents précités auxquels l'homme du métier pourra se référer.

Ce procédé est aussi caractérisé en ce que l'ester de l'acide (méth)acrylique est choisi parmi l'acrylate d'éthyle, l'acrylate de butyle, le méthacrylate de méthyle et leurs mélanges.

Ce procédé est aussi caractérisé en ce que le monomère contenant un groupement hydrophobe possède la formule générale : 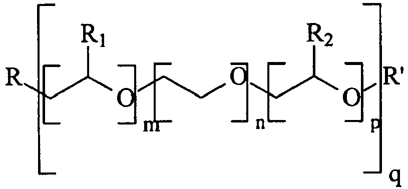 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

Ce procédé est aussi caractérisé en ce que le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

Ce procédé est aussi caractérisé en ce que l'émulsion aqueuse présente un extrait sec compris entre 10 % et 50 % en poids sec de polymère, par rapport à son poids total.

Ce procédé est aussi caractérisé en ce que l'émulsion présente une taille de particules comprise entre 50 nm et 500 nm.

Ce procédé est aussi caractérisé en ce que le polymère présente une masse molaire moyenne en poids comprise entre 20 000 g/mol et 1 000 000 g/mol.

Un autre objet de la présente invention consiste en une émulsion aqueuse directe, d'un polymère caractérisé en ce qu'il est constitué de, exprimé en % en poids de chacun des monomères :
a) 20 % à 60 % en poids d'acide méthacrylique et éventuellement d'acide acrylique,
b) 40 % à 80 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
   le total a) + b) + c) + d) étant égal à 100 %,
ou
a) 20 % à 60 % en poids d'acide méthacrylique et éventuellement d'acide acrylique,
b) 40 % à 80 % en poids d'au moins un ester de l'acide (méth)acrylique,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
   le total a) + b) + c) + d) + e) étant égal à 100 %,
et caractérisée en ce que ladite émulsion est exempte de tensio-actif et de solvant autre que l'eau.

Cette émulsion est aussi caractérisée en ce que le monomère contenant un groupement hydrophobe possède la formule générale : 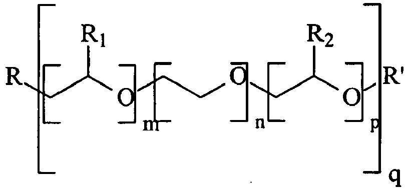 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

Cette émulsion est aussi caractérisée en ce que le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

Cette émulsion est aussi caractérisée en ce qu'elle présente un extrait sec compris entre 10 % et 50 % en poids sec de polymère, par rapport à son poids total.

Cette émulsion est aussi caractérisée en ce qu'elle présente une taille de particules comprise entre 50 nm et 500 nm.

Cette émulsion est aussi caractérisée en ce que le polymère présente une masse molaire moyenne en poids comprise entre 20 000 g/mol et 1 000 000 g/mol.

Un autre objet de la présente invention réside dans l'utilisation de l'émulsion précitée, comme agent épaississant d'une formulation aqueuse. Concrètement, l'émulsion est introduite dans le milieu à épaissir, dont on règle le pH au voisinage de la neutralité, afin d'obtenir l'effet d'épaississement. L'homme du métier, à partir d'expériences de routine, saura trouver le pH à partir duquel on observe le phénomène d'épaississement.

Cette utilisation est aussi caractérisée en ce que ladite formulation est choisie parmi une peinture aqueuse, une sauce de couchage papetière, une suspension aqueuse de matières minérales, un détergent, une formulation cosmétique, ou une formulation contenant un liant hydraulique.

Un dernier objet de la présente invention réside dans une formulation aqueuse contenant l'émulsion précitée, ladite formulation étant choisie parmi une peinture aqueuse, une sauce de couchage papetière, une suspension aqueuse de matières minérales, un détergent, une formulation cosmétique, ou contenant un liant hydraulique.

### EXEMPLES

### Exemple 1

Cet exemple concerne la synthèse de différentes émulsions de type ASE et HASE, sans tensio-actif et sans solvant autre que l'eau.

### Essai n° 1 selon l'invention

Cet essai concerne la fabrication d'une émulsion de type ASE, contenant un copolymère constitué de, exprimé en % en poids de chacun de ses monomères :
a) 36,5 % d'acide méthacrylique,
b) 62,3 % d'acrylate d'éthyle,
c) 1,2 % d'AMPS.

Dans un réacteur de 1 litre muni d'une agitation mécanique et d'un système de chauffage de type bain d'huile, on introduit 605 g d'eau bipermutée et 7,5 g d'une solution d'AMPS (solution à 55 % dans l'eau d'AMPS neutralisé par de la soude, commercialisé par la société LUBRIZOL™ sous le nom AMPS 2405).

On chauffe le milieu jusqu'au 82°C puis on introduit par un entonnoir le système catalytique constitué de 1,0 g de persulfate d'ammonium dissous dans 10g d'eau bipermutée et 0,1 g de métabisulfite de sodium dissous dans 10 g d'eau bipermutée.

On introduit ensuite, de manière continue et progressive 215 g d'acrylate d'éthyle et 140 g d'acide méthacrylique (à 90 %).
Pendant toute la durée de l'ajout, la température du milieu réactionnel est maintenue à 85°C (± 2).
Une fois l'ajout terminé, on rince la pompe avec 15 g d'eau bipermutée puis on laisse réagir pendant 30 minutes à 85°C (± 2).
On ajoute alors 0,15 g de persulfate d'ammonium dissous dans 20 g d'eau en 30 minutes tout en maintenant la température à 85°C (± 2) et on laisse réagir pendant 1 heure à 87°C (± 2).

On obtient alors une émulsion parfaitement homogène, contenant 34,0 % en poids de matière sèche, et dont la taille des particules, mesurée par Diffusion Dynamique de la Lumière est égale à 170 nm.

### Essais comparatifs

On a tout d'abord cherché à fabriquer le même polymère que dans l'essai 1, mais sans mettre en oeuvre d'AMPS, à savoir un polymère constitué de 36,5 % en poids d'acide méthacrylique et 63,5 % en poids d'acrylate d'éthyle.

Pour ce faire, on a mis en oeuvre le même protocole que précédemment, exception faite de l'introduction initiale d'AMPS. Après avoir laissé réagir à 85°C pendant 1 heure, on observe la formation d'un précipité trouble en solution, dont une partie se fixe au niveau de l'axe du réacteur (enrochement). La taille des particules formées est de l'ordre de 400 nm. Le milieu, très inhomogène et riche en particules de grosses tailles, ne se prête pas à des manipulations, et notamment à des opérations de pompage. Le stockage risque de plus de conduire à la sédimentation du produit.

On a ensuite mis en oeuvre une quantité d'AMPS supérieure à 22 % en poids de la masse totale des monomères engagés, selon un mode opératoire identique à celui décrit dans l'essai n° 1, le copolymère étant alors constitué, exprimé en % en poids de chacun de ses monomères :
a) 36,5 % d'acide méthacrylique,
b) 38,5 % d'acrylate d'éthyle,
c) 25,0 % d'AMPS.

On observe alors la formation d'espèces insolubles qui précipitent dans le milieu.

On a ensuite remplacé l'AMPS (1, 2 %, poids pour poids) par un autre monomère mis en oeuvre dans les émulsions ASE et HASE : soit de l'acide acrylique, soit de l'acide méthacrylique ou soit un ester qui est l'acrylate d'éthyle. On ne parvient pas à réaliser des émulsions homogènes ; les observations sont les mêmes que précédemment, avec la formation d'un précipité, le phénomène d'enrochement, et les conséquences néfastes qui s'en suivent.

On a ensuite substitué l'AMPS (toujours 1,2 % poids pour poids) par du styrène, du méthacrylate de lauryle, du méthacrylate de 2-sulfoéthyle, du styrène sulfonate de sodium, du sel de sodium du 1-allyloxy-2-hydroxypropyl sulfonate (Sipomer COPS 1) pour des résultats identiques (présence d'insolubles, et phénomène d'enrochement).

### Essais n° 2 à 10 selon l'invention

Les essais n° 2 à 10 concernent la synthèse d'autres émulsions illustrant l'invention, selon le même mode opératoire que décrit précédemment.

Les essais n° 2 à 7 illustrent d'autres compositions monomériques avec un taux massique d'AMPS fixé à 1,2 %, alors que les essais n° 8 à 10 illustrent d'autres taux en AMPS (on a alors maintenu constant le ratio massique entre l'acrylate d'éthyle et l'acide méthacrylique).

On obtient des émulsions parfaitement homogènes, dont les caractéristiques apparaissent dans le tableau 1.

**Tableau 1**

| **Essai n°** | **Composition monomérique (% massique)** | **Extrait sec (%)** | **Diamètre particule (nm)** |
|---|---|---|---|
| 1 | 1,2 AMPS / 62,3 AE / 36,5 AMA | 34,0 | 212 |
| 2 | 1,2 AMPS / 74,6 AE / 18,5 AA / 5,7 AMA | 32,3 | 214 |
| 3 | 1,2 AMPS / 68,7 AE / 18,6 AA / 11,5 AMA | 32,1 | 115 |
| 4 | 1,2 AMPS / 61,3 AE / 1,7 AA / 35,8 AMA | 32,1 | 198 |
| 5 | 1,2 AMPS / 60,3 AE / 3,3 AA / 35,2 AMA | 33,1 | 198 |
| 6 | 1,2 AMPS / 53,25 AE / 28,9 AA / 6,9 AMA / 9,75 MethC22OE25 | 38,5 | 183 |
| 7 | 1,2 AMPS / 62,2 AE / 28,5 AA / 3,3 AMA / 4,8 MethC22OE25 | 38,5 | 153 |
| 8 | 10 AMPS / 57,9 AE / 32,1 AMA | 34,0 | 220 |
| 9 | 15 AMPS / 55,4 AE / 29,6 AMA | 34,0 | 280 |
| 10 | 20 AMPS / 52,9 AE / 27,1 AMA | 34,0 | 319 |

Dans ce tableau, MethC22OE25 désigne un monomère de formule (I) dans laquelle R désigne la fonction méthacrylate, m=p=0, n=25, et R' désigne un groupe méthyle, AE désigne l'acrylate d'éthyle, AA et AMA désignent respectivement les acides acrylique et méthacrylique.

### Exemple 2

Cet exemple illustre le pouvoir épaississant des émulsions selon l'invention, mises en oeuvre dans de l'eau et de manière à avoir une teneur en matière active de 5 % en polymère sec.
Après introduction dans l'eau, le milieu est neutralisé par ajout de soude à un pH voisin de 6,5, et on mesure la viscosité Brookfield™ du milieu, à 25 °C et à 100 tours / minute, dont les valeurs sont reportées dans le tableau 2

**Tableau 2**

| **Emulsion selon l'essai n°** | **Viscosité Brookfield™ (mPa.s) du gel à 5 %** |
|---|---|
| 1 | 3 420 |
| 8 | 2 920 |
| 9 | 1 880 |
| 10 | 1 440 |

On démontre ainsi le caractère épaississant des émulsions fabriquées, dès l'instant où elles sont placées en solution aqueuse et dans des conditions alcalines.

### Exemple 3

Cet exemple illustre la mise en oeuvre, dans la formulation d'un béton, d'émulsions épaississantes de type ASE commerciales (contenant des tensio-actifs) et d'une émulsion selon l'invention exemptes de tensio-actifs.

Pour ce faire, on réalise un béton selon les techniques bien décrites dans la littérature, constitué de :
- 300 kg de ciment CEM I 52.5 N ;
- 880 de gros cailloux 10/20 ;
- 110 kg de sable 0/4 ;
dont le rapport eau sur ciment E / C est réglé à 0,5, et dans lequel on introduit, par rapport au poids sec de ciment :
- 1,23 % en poids en l'état d'un dispersant commercialisé par la société COATEX™ sous le nom Ethacryl™ 1030 ;
- 1 % en poids en l'état d'un antimousse commercialisé par la société HUNTSMANN™ sous le nom Empilan™ PF 7169.

Les essais A, B, C et D, mettent respectivement en oeuvre dans la formulation de béton 0,7 % en poids en l'état :
- d'une émulsion de type ASE contenant des tensio-actifs et commercialisée par la société COATEX™ sous le nom Viscoatex™ 730 ;
- d'une émulsion de type ASE contenant des tensio-actifs et commercialisée par la société COATEX™ sous le nom Rheocoat™ 35 ;
- d'une émulsion de type HASE contenant des tensio-actifs et commercialisée par la société COATEX™ sous le nom Rheocoat™ 66 ;
- d'une émulsion selon l'invention, qui est celle décrite dans l'essai n° 1.

Pour chacun des essais A, B, C et D, on a mesuré l'air occlus, selon la norme EN 12350-7. On obtient respectivement 9,5 %, 12,0 %, 12,5 % et 2,5 %.

L'émulsion épaississante selon l'invention permet donc de diminuer très sensiblement la quantité d'air introduite dans la formulation. On dispose donc au final d'un produit plus compact, à la résistance améliorée.

## Revendications

1. Procédé de fabrication en émulsion aqueuse directe d'un polymère, **caractérisé en ce que** ledit procédé met en jeu la réaction de polymérisation de, exprimé en % en poids de chacun des monomères :
a) 20 % à 60 % en poids d'acide méthacrylique et éventuellement d'acide acrylique,
b) 40 % à 80 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %,
ou
a) 20 % à 60 % en poids d'acide méthacrylique et éventuellement d'acide acrylique,
b) 40 % à 80 % en poids d'au moins un ester de l'acide (méth)acrylique,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
et **caractérisé en ce que** ledit procédé est exempt de tensio-actif et de solvant autre que l'eau.

2. Procédé selon la revendication 1, **caractérisé en ce que** l'ester de l'acide méthacrylique est choisi parmi l'acrylate d'éthyle, l'acrylate de butyle, le méthacrylate de méthyle et leurs mélanges.

3. Procédé selon l'une des revendications 1 ou 2, **caractérisé en ce que** le monomère contenant un groupement hydrophobe possède la formule générale (I) : 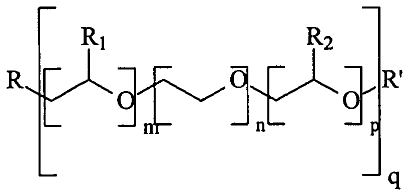 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

4. Procédé selon l'une des revendications 1 à 3, **caractérisé en ce que** le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

5. Procédé selon l'une des revendications 1 à 4, **caractérisé en ce que** l'émulsion aqueuse présente un extrait sec compris entre 10 % et 50 % en poids sec de polymère, par rapport à son poids total.

6. Procédé selon l'une des revendications 1 à 5, **caractérisé en ce que** l'émulsion présente une taille de particules comprise entre 50 nm et 500 nm, mesurée par diffusion dynamique de la lumière.

7. Procédé selon l'une des revendications 1 à 6, **caractérisé en ce que** le polymère présente une masse molaire moyenne en poids comprise entre 20 000 g/mol et 1 000 000 g/mol.

8. Emulsion aqueuse directe, d'un polymère **caractérisé en ce qu'**il est constitué de, exprimé en % en poids de chacun des monomères :
a) 20 % à 60 % en poids d'acide méthacrylique et éventuellement d'acide acrylique,
b) 40 % à 80 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %,
ou
a) 20 % à 60 % en poids d'acide méthacrylique et éventuellement d'acide acrylique,
b) 40 % à 80 % en poids d'au moins un ester de l'acide (méth)acrylique,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
et **caractérisée en ce que** ladite émulsion est exempte de tensio-actif et de solvant autre que l'eau.

9. Emulsion selon la revendication 8, **caractérisée en ce que** le monomère contenant un groupement hydrophobe possède la formule générale (I) : 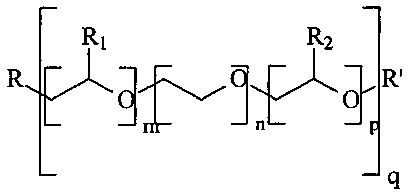 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

10. Emulsion selon l'une des revendications 8 ou 9, **caractérisée en ce que** le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

11. Emulsion selon l'une des revendications 8 à 10, **caractérisée en ce qu'**elle présente un extrait sec compris entre 10 % et 50 % en poids sec de polymère, par rapport à son poids total.

12. Emulsion selon l'une des revendications 8 à 11, **caractérisée en ce qu'**elle présente une taille de particules comprise entre 50 nm et 500 nm, mesurée par diffusion dynamique de la lumière.

13. Emulsion selon l'une des revendications 8 à 12, **caractérisée en ce que** le polymère présente une masse molaire moyenne en poids comprise entre 20 000 g/mol et 1 000 000 g/mol.

14. Utilisation de l'émulsion selon l'une des revendications 8 à 13, comme agent épaississant d'une formulation aqueuse.

15. Utilisation selon la revendication 14, **caractérisée en ce que** ladite formulation est choisie parmi une peinture aqueuse, une sauce de couchage papetière, une suspension aqueuse de matières minérales, un détergent, une formulation cosmétique, ou contenant un liant hydraulique.

16. Formulation aqueuse contenant l'émulsion selon l'une des revendications 8 à 13.

17. Formulation selon la revendication 16, **caractérisée en ce qu'**elle est choisie parmi une peinture aqueuse, une sauce de couchage papetière, une suspension aqueuse de matières minérales, un détergent, une formulation cosmétique, ou une formulation contenant un liant hydraulique.

## Patentansprüche

1. Verfahren zur Herstellung eines Polymers in direkter wässriger Emulsion, **dadurch gekennzeichnet, dass** bei dem Verfahren die folgenden Stoffe eine Polymerisationsreaktion erfähren, ausgedrückt in Gewichts-% jedes der Monomere:
a) 20 bis 60 Gewichts-% an Methacrylsäure und möglicherweise Acrylsäure,
b) 40 bis 80 Gewichts-% mindestens eines (Meth)acrylsäureesters,
c) 0,05 bis 22 Gewichts-% an 2-Acrylamido-2-methylpropansulfonsäure,
d) 0 bis 1 Gewichts-% mindestens eines vernetzenden Monomers,
wobei a) +b) + c) + d) zusammengenommen gleich 100 % sind,
oder
a) 20 bis 60 Gewichts-% an Methacrylsäure und möglicherweise an Acrylsäure,
b) 40 bis 80 Gewichts-% mindestens eines (Meth)acrylsäureesters,
c) 0,5 bis 25 Gewichts-% eines Monomers, welches eine hydrophobe Gruppe enthält,
d) 0,05 bis 22 Gewichts-% an 2-Acrylamido-2-methylpropansulfonsäure,
e) 0 bis 1 Gewichts-% mindestens eines vernetzenden Monomers,
wobei a) +b) + c) + d) + e) zusammengenommen gleich 100 % sind,
und **dadurch gekennzeichnet, dass** das Verfahren ohne Tensid und ohne Lösemittel außer Wasser auskommt.

2. Verfahren nach Anspruch 1, **dadurch gekennzeichnet, dass** der Methacrylsäureester aus Ethylacrylat, Butylacrylat, Methylmethacrylat und deren Mischungen ausgewählt ist.

3. Verfahren nach einem der Ansprüche 1 oder 2, **dadurch gekennzeichnet, dass** das Monomer, welches eine hydrophobe Gruppe enthält, die allgemeine Formel (I) hat: 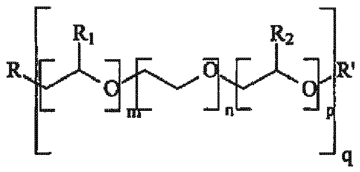 wobei:
- m, n, p und q ganze Zahlen sind und m, n, p kleiner als 150 sind, q größer als 0 ist und mindestens eine der ganzen Zahlen m, n und p von Null verschieden ist,
- R eine polymerisierbare funktionelle Vinylgruppe aufweist,
- R₁ und R₂ identisch oder verschiedenartig sind und Wasserstoffatome oder Alkylgruppen darstellen,
R' eine hydrophobe Gruppe ist, die mindestens 6 und höchstens 36 Kohlenstoffatome aufweist.

4. Verfahren nach einem der Ansprüche 1 bis 3, **dadurch gekennzeichnet, dass** das vernetzende Monomer aus Ethylenglycoldimethacrylat, Trimethylolpropantriacrylat, Diallylphthalate, Allylacrylat, den Allylmaleaten, Methylen-bis-acrylamid, Methylen-bis-methacrylamid, Tetrallyloxyethan, den Triallylcyanuraten, den Allylethern, welche ausgehend von Polyolen erhalten werden, ausgewählt ist.

5. Verfahren nach einem der Ansprüche 1 bis 4, **dadurch gekennzeichnet, dass** die wässrige Emulsion einen Trockenextrakt im Bereich von 10 bis 50 Gewichts-% an trockenem Polymer aufweist, bezogen auf ihr Gesamtgewicht.

6. Verfahren nach einem der Ansprüche 1 bis 5, **dadurch gekennzeichnet, dass** die Emulsion eine Partikelgröße im Bereich von 50 nm bis 500 nm aufweist, gemessen mittels dynamischer Lichtstreuung.

7. Verfahren nach einem der Ansprüche 1 bis 6, **dadurch gekennzeichnet, dass** das Polymer ein Gewichtsmittel des Molekulargewichts im Bereich von 20.000 g/mol und 1.000.000 g/mol aufweist.

8. Direkte wässrige Emulsion eines Polymers, welches **dadurch gekennzeichnet ist, dass** es aus Folgendem besteht, ausgedrückt in Gewichts-% jedes der Monomere:
a) 20 bis 60 Gewichts-% an Methacrylsäure und möglicherweise an Acrylsäure,
b) 40 bis 80 Gewichts-% mindestens eines (Meth)acrylsäureesters,
c) 0,05 bis 22 Gewichts-% an 2-Acrylamido-2-methylpropansulfonsäure,
d) 0 bis 1 Gewichts-% mindestens eines vernetzenden Monomers,
wobei a) +b) + c) + d) zusammengenommen gleich 100 % sind,
oder
a) 20 bis 60 Gewichts-% an Methacrylsäure und möglicherweise an Acrylsäure,
b) 40 bis 80 Gewichts-% mindestens eines (Meth)acrylsäureesters,
c) 0,5 bis 25 Gewichts-% eines Monomers, welches eine hydrophobe Gruppe enthält,
d) 0,05 bis 22 Gewichts-% 2-Acrylamido-2-methylpropansulfonsäure,
e) 0 bis 1 Gewichts-% mindestens eines vernetzenden Monomers,
wobei a) +b) + c) + d) + e) zusammengenommen gleich 100 % sind,
und **dadurch gekennzeichnet, dass** die Emulsion frei von Tensiden und Lösemitteln außer Wasser ist.

9. Emulsion nach Anspruch 8, **dadurch gekennzeichnet, dass** das Monomer, welches eine hydrophobe Gruppe enthält, die allgemeine Formel (I) hat: 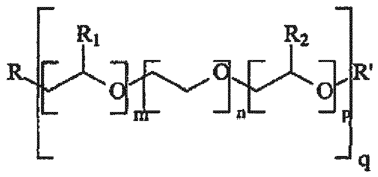 wobei:
- m, n, p und q ganze Zahlen sind und m, n, p kleiner als 150 sind, q größer als 0 ist und mindestens eine der ganzen Zahlen m, n und p von Null verschieden ist,
- R eine polymerisierbare funktionelle Vinylgruppe aufweist,
- R₁ und R₂ identisch oder verschiedenartig sind und Wasserstoffatome oder Alkylgruppen darstellen,
- R' eine hydrophobe Gruppe ist, die mindestens 6 und höchstens 36 Kohlenstoffatome aufweist.

10. Emulsion nach einem der Ansprüche 8 oder 9, **dadurch gekennzeichnet, dass** das vernetzende Monomer aus Ethylenglycoldimethacrylat, Trimethylolpropantriacrylat, Diallylphthalate, Allylacrylat, den Allylmaleaten, Methylen-bis-acrylamid, Methylen-bis-methacrylamid, Tetrallyloxyethan, den Triallylcyanuraten, den Allylethern, welche ausgehend von Polyolen erhalten werden, ausgewählt ist.

11. Emulsion nach einem der Ansprüche 8 bis 10, **dadurch gekennzeichnet, dass** sie einen Trockenextrakt im Bereich von 10 bis 50 Gewichts-% an trockenem Polymer aufweist, bezogen auf ihr Gesamtgewicht.

12. Emulsion nach einem der Ansprüche 8 bis 11, **dadurch gekennzeichnet, dass** sie eine Partikelgröße im Bereich von 50 nm bis 500 nm aufweist, gemessen mittels dynamischer Lichtstreuung.

13. Emulsion nach einem der Ansprüche 8 bis 12, **dadurch gekennzeichnet, dass** das Polymer ein Gewichtsmittel des Molekulargewichts im Bereich von 20.000 g/mol und 1.000.000 g/mol aufweist.

14. Verwendung der Emulsion nach einem der Ansprüche 8 bis 13, als Verdickungsmittel für eine wässrige Formulierung.

15. Verwendung nach Anspruch 14, **dadurch gekennzeichnet, dass** die Formulierung aus einem wässrigen Anstrichmittel, einer Papierstreichmasse, einer wässrigen Suspension mineralischer Stoffe, einem Reinigungsmittel, einer kosmetischen Formulierung, oder einer solchen, die ein hydraulisches Bindemittel enthält, ausgewählt ist.

16. Wässrige Formulierung, welche die Emulsion nach einem der Ansprüche 8 bis 13 enthält.

17. Formulierung nach Anspruch 16, **dadurch gekennzeichnet, dass** sie aus einem wässrigen Anstrichmittel, einer Papierstreichmasse, einer wässrigen Suspension mineralischer Stoffe, einem Reinigungsmittel, einer kosmetischen Formulierung, oder einer Formulierung, die ein hydraulisches Bindemittel enthält, ausgewählt ist.

## Claims

1. A method for manufacturing a polymer in direct aqueous emulsion, **characterized in that** said method uses the polymerization reaction of, expressed as a % by weight of each of the monomers:
a) 20% to 60% by weight of methacrylic acid, and potentially acrylic acid,
b) 40% to 80% by weight of at least one ester of (meth)acrylic acid,
c) 0.05% to 22% by weight of 2-acrylamido-2-methylpropane sulfonic acid,
d) 0 to 1% by weight of at least one cross-linked monomer,
the total a) + b) + c) + d) being equal to 100%,
or
a) 20% to 60% by weight of methacrylic acid, and potentially acrylic acid,
b) 40% to 80% by weight of at least one ester of (meth)acrylic acid,
c) 0.5% to 25% by weight of the monomer containing a hydrophobic group,
d) 0.05% to 22% by weight of 2-acrylamido-2-methylpropane sulfonic acid,
e) 0 1% by weight of at least one cross-linked monomer,
the total a) + b) + c) + d) + e) being equal to 100%,
and **characterized in that** said method is free of surfactants and solvents other than water.

2. A method according to claim 1, **characterized in that** the ester of (meth)acrylic acid is chosen from among ethyl acrylate, butyl acrylate, methyl methacrylate, and mixtures thereof.

3. A method according to one of the claims 1 or 2, **characterized in that** the monomer containing a hydrophobic group possesses the general formula (I): 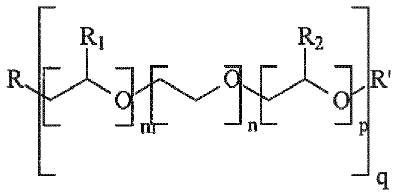 where:
- m, n, p and q are integers and m, n, p are less than 150, q is greater than 0, and at least one integer among m, n and p is nonzero,
- R has a polymerizable vinylic function,
- R₁ and ₂ are identical or different, and represent hydrogen atoms or alkyl groups,
- R' is a hydrophobic group comprising at least 6 and at most 36 carbon atoms.

4. A method according to one of the claims 1 to 3, **characterized in that** the cross-linked monomer is chosen from ethylene glycol dimethacrylate, trimethylolpropanetriacrylate, diallyl phthalate, allyl acrylate, allyl maleates, methylene-bis-acrylamide, methylene-bismethacrylamide, tetrallyloxyethane, triallylcyanurates, and allylic ethers obtained from polyols.

5. A method according to one of the claims 1 to 4, **characterized in that** the aqueous emulsion exhibits a solids content of between 10% and 50% by dry weight of polymer, in relation to its total weight.

6. A method according to one of the claims 1 to 5, **characterized in that** the aqueous emulsion exhibits a particle size between 50 and 500 nm, measured by dynamic light scattering.

7. A method according to one of the claims 1 to 6, **characterized in that** the polymer exhibits an average molar mass by weight of between 20,000 g/mol and 1,000,000 g/mol.

8. A direct aqueous emulsion of a polymer **characterized in that** it is made up, express as a % by weight of each of the monomers:
a) 20% to 60% by weight of methacrylic acid, and potentially acrylic acid,
b) 40% to 80% by weight of at least one ester of (meth)acrylic acid,
c) 0.05% to 22% by weight of 2-acrylamido-2-methylpropane sulfonic acid,
d) 0 to 1% by weight of at least one cross-linked monomer,
the total a) + b) + c) + d) being equal to 100%,
or
a) 20% to 60% by weight of methacrylic acid, and potentially acrylic acid,
b) 40% to 80% by weight of at least one ester of (meth)acrylic acid,
c) 0.5% to 25% by weight of the monomer containing a hydrophobic group,
d) 0.05% to 22% by weight of 2-acrylamido-2-methylpropane sulfonic acid,
e) 0 to 1% by weight of at least one cross-linked monomer,
the total a) + b) + c) + d) + e) being equal to 100%,
and **characterized in that** said emulsion is free of surfactants and solvents other than water.

9. An emulsion according to claim 8, **characterized in that** said monomer containing one hydrophobic group has the general formula (I): 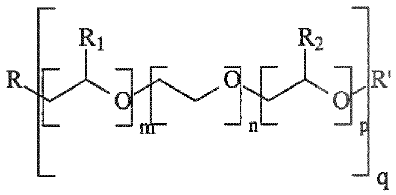 where:
- m, n, p and q are integers and m, n, p are less than 150, q is greater than 0, and at least one integer among m, n and p is nonzero,
- R has a polymerizable vinylic function,
- R₁ and R₂ are identical or different, and represent hydrogen atoms or alkyl groups,
- R' is a hydrophobic group comprising at least 6 and at most 36 carbon atoms.

10. An emulsion according to one of the claims 8 or 9, **characterized in that** the cross-linked monomer is chosen from ethylene glycol dimethacrylate, trimethylolpropanetriacrylate, diallyl phthalate, allyl acrylate, allyl maleates, methylene-bis-acrylamide, methylene-bis-methacrylamide, tetrallyloxyethane, triallylcyanurates, and allylic ethers obtained from polyols.

11. An emulsion according to one of the claims 8 to 10, **characterized in that** it exhibits a solids content of between 10% and 50% by dry weight of polymer, in relation to its total weight.

12. An emulsion according to one of the claims 8 to 11, **characterized in that** it exhibits a particle size between 50 nm and 500 nm, measured by dynamic light scattering.

13. An emulsion according to one of the claims 8 to 12, **characterized in that** the polymer exhibits an average molar mass by weight of between 20,000 g/mol and 1,000,000 g/mol.

14. The use of the emulsion according to one of the claims 8 to 13, as a thickening agent of an aqueous formulation.

15. The use according to claim 14, **characterized in that** said formulation is chosen from among an aqueous paint, a paper coating color, an aqueous suspension of mineral materials, a detergent, a cosmetic formulation, or containing a hydraulic binder.

16. An aqueous formulation containing the emulsion according to one of the claims 8 to 13.

17. A formulation according to claim 16, **characterized in that** it is chosen from among a water-based paint, a paper coating color, an aqueous suspension of mineral materials, a detergent, a cosmetic formulation, or a formulation containing a hydraulic binder.
